# EUROPEAN PATENT APPLICATION

(11) **EP 3 581 237 A1**
(43) Date of publication of application: **18.12.2019**
(21) Application number: 18178092.5
(22) Date of filing: 15.06.2018
(51) Int. Cl.: A61M 39/10

(54) **CONNECTOR FOR CONNECTING ENTERAL FEEDING DEVICES**

(71) Applicant: Cedic S.r.l., 20068 Peschiera Borromeo (MI) (IT)
(72) Inventor: Bronzato, Luca, 37024 (VR) Negrar (IT)
(74) Representative: Caspary, Karsten

(57) **Abstract**

A connector (1) for connecting enteral feeding equipment is provided comprising: a first section (3) having a first port configured to receive a first connecting element (5) of a syringe or a tubing system (7), the first port comprising an axial protrusion (9) with a first bore opening (11) surrounded by a recess (13), the recess (13) adapted to receive a thread portion (15) of the first connecting element (5), a second section (17) having a second port configured to be directly connected to a low profile gastrostomy device (21) or a gastrostomy button, a transition section (23) between the first and the second section (17), and a bore (25) extending centrally through the connector (1) and having an inner diameter enabling flow of liquid between the first port and the second port, wherein the second section (17) comprises a second bore opening (19) and a single radial protrusion (27) of predetermined width extending from the outer surface (28) of the second section (17) and being located at a predetermined distance from the second bore opening (19), such that the connection between the second port of the connector (1) and the low profile gastrostomy button (21) is configured to be tubeless and locked by twisting.

## Description

The present invention relates to a connector for connecting enteral feeding equipment and particularly to a connector configured to attach a syringe or a tubing system with a low profile gastrostomy device.

Patients whose capability to take food orally by their own abilities is deteriorated due to aging or diseases are generally fed with fluid substances such as liquid food or nutrient preparation or formula by using a gastrostomy device. Such a gastrostomy device can be a low profile gastrointestinal feeding system which usually includes a feeding set having a nutrition source attached to one end and a low profile gastrostomy tube connected to the other end. The low profile gastrostomy tube is normally inserted through an established, mature stoma extending through the patients abdominal and stomach walls. The tube is held in place by an internal retention member such as an inflatable balloon or other retention means deployed inside a patient's stomach or other visceral organ to anchor the free end of the gastrostomy tube to the organ.

The low profile gastrostomy device is also called gastrostomy button which comprises a relatively flat surface and a bore opening perpendicular to the skin surface. In the prior art the gastrostomy button is usually used in connection with so-called extension sets to provide a connection to enteral feeding sets or syringes. The extension sets comprise a right angle or L-geometry connector at their distal end, approximately 30 cm of PVC tubing having an on/off clamp and a Y-port at the proximal end for a flexible connection with nutrition sources. The extension set allows to perform administration of enteral nutrition in the form of continuous and bolus feeding and also to measure residual inside the stomach and to decompress or vent the stomach.

An example of a low profile gastrostomy device is described in US 2009/0287156 A1.

Due to hygienic reasons these extension sets are disposable and must be replaced regularly, usually every few weeks. After every feeding procedure, the extension set must be cleaned with warm soapy water and rinsed thoroughly. This contributes to a high water consumption and also to a high waste of plastic pieces. Furthermore, an extension set comprises a certain number of molded parts, tubing and the assembly of these components.

It is therefore the object of the present invention to provide a connector for connecting enteral feeding equipment which is less complex than an enteral extension set, which reduces the costs compared with an extension set, which is easy to use and comprises at least all the functionality of an enteral extension set including attachment safety and avoidance of misconnections.

This object is achieved by the subject matter of claim 1 of the present invention. Advantageous embodiments are described in the dependent claims.

According to the invention, a connector for connecting enteral feeding equipment is provided comprising a first section having a first port configured to receive a first connecting element of a syringe or a tubing system, the first port comprising an axial protrusion with a first bore opening surrounded by a recess, the recess adapted to receive a thread portion of the first connecting element, a second section having a second port configured to be directly connected to a low profile gastrostomy device or a gastrostomy button, a transition section between the first and the second section, and a bore extending centrally through the connector and having an inner diameter enabling flow of liquid between the first port and the second port, wherein the second section comprises a second bore opening and a single radial protrusion of predetermined width extending from the outer surface of the second section and being located at a predetermined distance from the second bore opening, such that the connection between the second port of the connector and the low profile gastrostomy device is configured to be tubeless and locked by twisting. Such a connector reduces the costs significantly because the extension set hither to used can be dispensed with. The connector allows for a direct connection without any tubing between an enteral feeding set or a syringe which are compatible with the first port of the connector according to the invention. By replacing the extension set with the single connector of the invention, costs are reduced and also waste of deducted because the connector according to the invention uses less material, less production steps and requires less procedural steps for the enteral feeding process. By using a small and single component instead of an extension set having a number of components, the ease of use of the entire enteral feeding process is also increased because it only requires the connection of the connector to the gastrostomy button/device and on the other end to a syringe or a corresponding tube connector. The tedious step of washing all the components of the extension set is, for example, avoided because the connector according to the invention can easily be replaced. Alternatively, washing of the connector is much less cumbersome than washing the entire extension set.

In addition to the above mentioned advantages the connector according to the invention fulfils the requirements of providing a safe environment in the sense that it is only compatible with the corresponding counter parts, i. e. the special configuration of the bore opening of the gastrostomy button and the male connection at the other end.

It is preferred that the first connector is a male connector according to the ISO 80369-3 standard and in that the low profile gastrostomy device accords to industrial standard. By fulfilling these requirements the chance of misconnections with other medical connections, IV sets or the like is reduced to a minimum.

Preferably, the outer diameter of the first section is larger than the outer diameter of the second section. This improves the handling of the connector and increases its stability.

It is further preferred that the outer surface of the first bore opening is tapered. This enables an easier connection with the corresponding connection peace. Advantageously, the outer surface of the second section comprises a tapered portion. This facilitates the introduction of the second section into the corresponding bore opening of the gastrostomy button.

It is further preferred that the tapered portion extends from the second bore opening by a predetermined distance.

Preferably, the connector comprises a radially extending flange arranged on the second section near the transition section. This flange can serve as a stop for the insertion of the connector into the gastrostomy device and thus eases the handling of the connector according to the invention.

With further advantage, the connector comprises a circumferential annular channel configured as a strap seat arranged on the first section near the transition section. This annular channel or strap seat can hold a circular loop peace which is configured to comprise a cap for either one of the first and second bore openings of the connector according to the invention. In the same fashion a label can be attached and fixed to this annular channel.

Preferably, a radial recess is located on the second section adjacent to the radial protrusion. Such radial recess marks the beginning of the tapered portion towards the second bore opening and is configured to receive a complementary protrusion located in the bore opening of the gastrostomy button.

It is further preferred that the flange is at an axial position between the radial protrusion and the transition section and comprises an outer diameter larger than the outer diameter of the second section and smaller than the outer diameter of the first section.

It is further preferred that the inner diameter of the bore is not constant along its length. It can be tapered from a larger first bore opening to a smaller second bore opening or vice versa.

With further advantage the first section comprises a grip portion on its outer surface which eases the handling of the connector according to the invention.

The present invention will now be explained with reference to the accompanying drawings in which:
Fig. 1 is a perspective view of a preferred embodiment of the connector according to the invention;
Fig. 2 is a side elevation view in cross section of the preferred embodiment shown in Fig. 1;
Fig. 3 is a side elevation view of the preferred embodiment shown in Fig. 1;
Fig. 4 is a side elevation view of the connector according to the invention shown in Fig. 3 rotated by 90 degrees;
Fig. 5 is an exploded perspective view of the preferred embodiment of the connector according to the invention shown with a gastrostomy button and an enteral feeding tube; and
Fig. 6 is a further exploded perspective view of the components shown in Fig. 5.

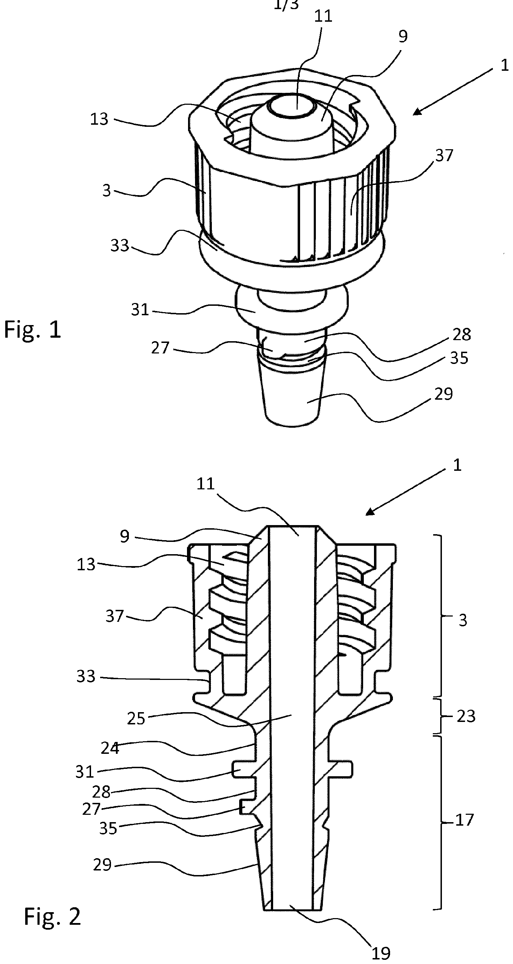

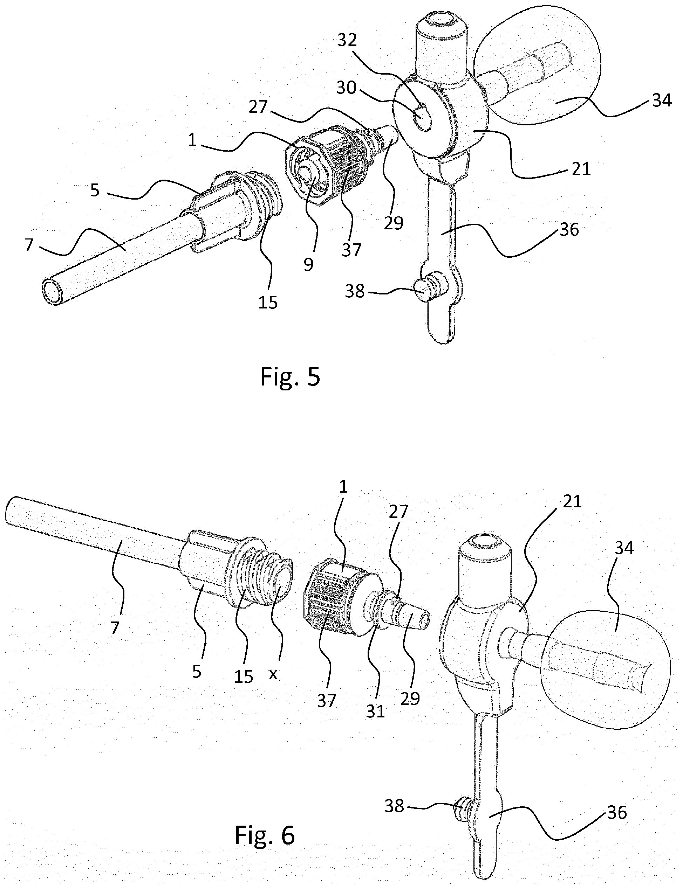

Figs. 1 to 4 show different views of the connector according to the invention according to a preferred embodiment. The connector 1 comprises a first section 3, a transition section 23 and a second section 17 which are arranged from top to bottom as can be seen in Fig. 2, 3 and 4. The first section 3 includes a first port configured to receive a first connector of a syringe or a tubing system (which are shown in Figs. 5 and 6). The first port comprises an axial protrusion 9 with a first bore opening 11 which is surrounded by a recess 13, wherein the recess 13 is adapted to receive a thread portion of a first connector. The inner surface of the outer wall of the first port thus comprises a thread, and the axial protrusion 9 has a slightly tapered outer surface which interacts with a corresponding recess of a first connector. On the outside of the first port there is a grip portion 37 which ensures that the connector 1 can be held tight when screwing the respective first connector with the connector 1.

It can be seen in this preferred embodiment that the upper outer edge of the first port for usability reasons has a substantially octagonal shape, which could also be fully circular or hexagonal or any other polygonal shape.

Close to the transition section 23 the first section 3 comprises a circumferential annular channel 33 which is configured as a strap seat to accommodate for rings which can be attached to labels or other components such as lids, covers and the like. The depth and width of the circumferential annular channel 33 are sufficiently dimensioned so that a corresponding ring can be attached in a press fit.

The transition section 23 is configured as a tapered section and connects the first section 3 having a larger outer diameter with the second section 17 comprising a smaller diameter. The second section 17 starts at the transition section 23 with a cylindrical portion 24 which ends at a radially extending flange 31 having a circular outer diameter which is significantly larger than the outer diameter of the cylindrical portion 24 and serves as a stop as will be explained further below.

Adjacent to the radially extending flange 31 is a cylindrical outer surface 28 which, at a predetermined distance, comprises a single radial protrusion 27 of a predetermined width extending from the outer surface 28 slightly less than the radially extending flange 31. This single radial protrusion 27 corresponds to a respective recess in the low profile gastrostomy device such that the connection between the connector 1 and the gastrostomy device can be locked by twisting as will be explained further below. In cross section, the radial protrusion 27 preferably comprises a rectangular shape but may optionally include rounded edges. Alternatively, the radial protrusion 27 may have a semicircular cross section in a plane perpendicular to the connector axis.

Adjacent to the radial protrusion 27 towards the second end of the connector 1 is a circular radial recess 35. At the second end, the connector 1 comprises a tapered portion 29. The lower end of the tapered portion 29, i. e. the portion with the smallest outer diameter marks the end of the second section 17 with the second bore opening 19.

A bore 25 extends centrally along the axis of the connector 1 between the first bore opening 11 and the second bore opening 19. The shape of the bore 25 is preferably cylindrical but may optionally comprise at least one tapered portion.

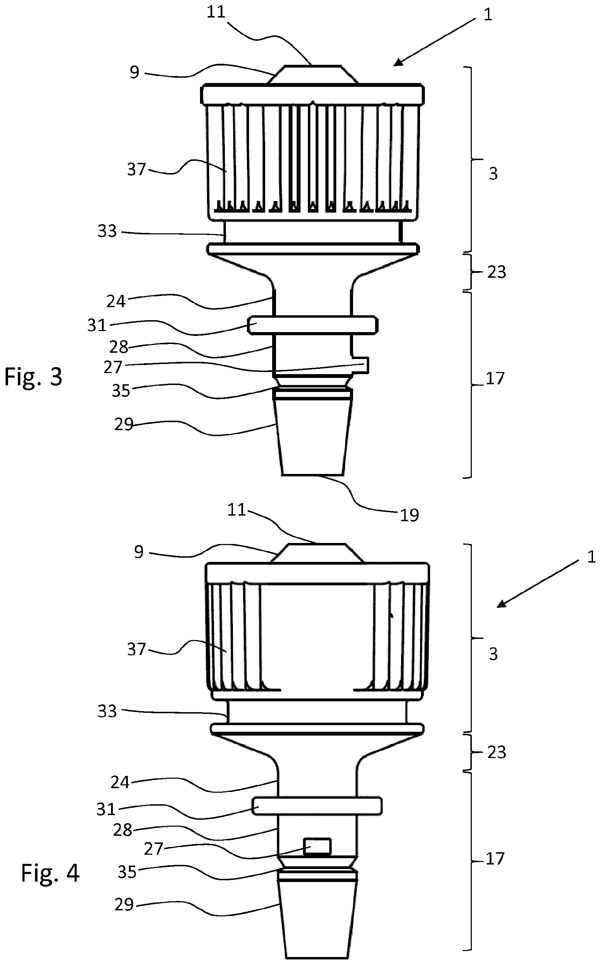

Figs. 2, 3 and 4 show the connector 1 viewed from different directions: it can be seen that in Fig. 2, the radial protrusion 27 is on the left hand side, whereas in Fig. 3 it is located on the right. The view in Fig. 4 is central, so that the radial protrusion 27 extends perpendicular to the drawing plane. In the shown embodiment, the width of the radial protrusion 27 is larger than its height (dimension along the connector axis).

In the shown embodiment the first section comprises a port configured as a male ENfit port according to ISO standard 80369-3 which relates to small-bore connectors for enteral medical devices. This standard has been developed because of several incidents, with catastrophic consequences, resulting from firstly, the administration of inappropriate medication into the alimentary canal and secondly, from enteral solutions being administered via incorrect routes, including intravenously and into the airway. Thus, it represents the industrial standard with a maximum level of connection safety.

The function of the connector according to the invention will now be described with reference to Figs. 5 and 6. Both Figs. show the connector 1 in an exploded view between a tubing system 7 having a first connector 5 comprising female connecting port with an outer thread portion 15. The female connecting port is the supplementary port to the male first port of the first section 3 of the connector according to the invention. The connection between the respective connectors is performed via a known twist-and-lock mechanism which is commonly known in the field of medical connectors. It must be noted that instead of a tubing system 7, a syringe or any other enteral feeding equipment with the respective female connector could be used.

The second section 17 of the connector 1 with the tapered section 29 at its end is configured to be directly connected with a low profile gastrostomy device 21, also called gastrostomy button shown on the right hand side in Figs. 5 and 6. The gastrostomy device 21 comprises for that purpose an opening 30 having a recess 32 on its perimeter. A balloon 34 is schematically shown on the other side of the main body of the gastrostomy device 21 which in use is located inside the patient, i.e. underneath the epidermis, to hold the gastrostomy device 21 in place. Arranged at the side of the body of the gastrostomy device 21 is a strap 36 which near its free end comprises a cover pin 38 which is configured to seal the opening 30 when no feeding equipment is mounted.

The structure of the three components leaves it open which of the respective components are first connected with each other. Since the connection of the first connector of the tubing system with the connector according to the invention has in principle been described above, the connection of the connector 1 with the gastrostomy device 21 is explained in detail: firstly, the tapered section 29 of the connector 1 is inserted into the opening 30 of the gastrostomy device 21 so that the single radial protrusion 27 is aligned with the recess 32. A further insertion of the connector 1 into the opening 30 under consideration of the shape of the protrusion 27 matching with the shape of the recess 32 will enable the rotation of the connector 1 with its second section 17 partially inside the body of the gastrostomy device 21. Upon rotating the connector 1 with respect to the body in the clockwise direction, the protrusion 27 will engage with a corresponding element so that a press fit is achieved and the connector 1 is firmly held in place on the gastrostomy device 21. In this position, the enteral feeding may take place. The engagement is detachable so that a short twist of the connector 1 in the counter-clockwise direction will bring the recess 30 in alignment with the protrusion 27 which enables an axial displacement and disengagement of the two components.

With the subject matter of the present invention a connector for connecting enteral feeding equipment is provided which is less complex than an enteral extension set, which reduces the costs compared with an extension set, which is easy to use and which comprises at least all the functionality of an enteral extension set including attachment safety and avoidance of misconnections.

## Claims

1. Connector (1) for connecting enteral feeding equipment comprising:
a first section (3) having a first port configured to receive a first connecting element (5) of a syringe or a tubing system (7), the first port comprising an axial protrusion (9) with a first bore opening (11) surrounded by a recess (13), the recess (13) adapted to receive a thread portion (15) of the first connecting element (5),
a second section (17) having a second port configured to be directly connected to a low profile gastrostomy device (21) or a gastrostomy button,
a transition section (23) between the first and the second section (17), and
a bore (25) extending centrally through the connector (1) and having an inner diameter enabling flow of liquid between the first port and the second port,
wherein the second section (17) comprises a second bore opening (19) and a single radial protrusion (27) of predetermined width extending from the outer surface (28) of the second section (17) and being located at a predetermined distance from the second bore opening (19), such that the connection between the second port of the connector (1) and the low profile gastrostomy button (21) is configured to be tubeless and locked by twisting.

2. Connector (1) according to claim 1, **characterized in that** the first connecting element (5) is a male connector according to the ISO 80369-3 standard and **in that** the low profile gastrostomy device (21) accords to industrial standard.

3. Connector (1) according to one of the previous claims, **characterized in that** the outer diameter of the first section (3) is larger than the outer diameter of the second section (17).

4. Connector (1) according to one of the previous claims, **characterized in that** the outer surface of the first bore opening (11) is tapered.

5. Connector (1) according to one of the previous claims, **characterized in that** the outer surface of the second section (17) comprises a tapered portion (29).

6. Connector (1) according to claim 5, **characterized in that** the tapered portion (29) extends from the second bore opening (19) by a predetermined distance.

7. Connector (1) according to one of the previous claims, **characterized in that** it comprises a radially extending flange (31) arranged on the second section (17) near the transition section (23).

8. Connector (1) according to one of the previous claims, **characterized in that** it comprises a circumferential annular channel (33) configured as a strap seat arranged on the first section (3) near the transition section (23).

9. Connector (1) according to one of the previous claims, **characterized in that** a radial recess (13) is located on the second section (17) adjacent to the radial protrusion (27).

10. Connector (1) according to claim 7, **characterized in that** the flange (33) is at an axial position between the protrusion (27) and the transition section (23) and comprises an outer diameter larger than the outer diameter of the second section (17) and smaller than the outer diameter of the first section (3).

11. Connector (1) according to one of the previous claims, **characterized in that** the inner diameter of the bore (25) is not constant along its length.

12. Connector (1) according to one of the previous claims, **characterized in that** the first section (3) comprises a grip portion (37).
